# EUROPÄISCHE PATENTSCHRIFT

(11) **EP 3 621 664 B1**
(45) Veröffentlichungstag und Bekanntmachung des Hinweises auf die Patenterteilung: **04.05.2022**
(21) Anmeldenummer: 19718401.3
(22) Anmeldetag: 23.04.2019
(51) Int. Cl.: A61L 27/04, A61L 27/08, A61L 27/42, A61L 27/58, A61L 31/02, A61L 31/12, A61L 31/14

(54) **RESORBIERBARES IMPLANTATMATERIAL AUS MAGNESIUM ODER EINER MAGNESIUMLEGIERUNG**
RESORBABLE IMPLANT MATERIAL MADE OF MAGNESIUM OR A MAGNESIUM ALLOY
MATÉRIAU D'IMPLANT RÉSORBABLE DE MAGNÉSIUM OU D'ALLIAGE DE MAGNÉSIUM

(30) Priorität: 03.05.2018 EP 18170669
(43) Veröffentlichungstag der Anmeldung: 18.03.2020
(73) Patentinhaber: Helmholtz-Zentrum hereon GmbH, 21502 Geesthacht (DE)
(72) Erfinder: DIERINGA, Hajo, 21335 Lüneburg (DE)
(74) Vertreter: Uexküll & Stolberg
(86) Internationale Anmeldenummer: PCT/EP2019/060326
(87) Internationale Veröffentlichungsnummer: WO 2019/211121

(56) Entgegenhaltungen:
- WO-A1-2010/110919
- WO-A1-2015/144439
- WO-A2-2013/158869
- GONG HAIBO ET AL: "Fabrication, biodegradation behavior and cytotoxicity of Mg-nanodiamond composites for implant application", JOURNAL OF MATERIALS SCIENCE: MATERIALS IN MEDICINE, SPRINGER NEW YORK LLC, UNITED STATES, Bd. 26, Nr. 2, 11. Februar 2015 (2015-02-11), Seiten 1-9, XP035468029, ISSN: 0957-4530, DOI: 10.1007/S10856-015-5441-3 [gefunden am 2015-02-11]
- ZHANG Q ET AL: "Fluorescent PLLA-nanodiamond composites for bone tissue engineering", BIOMATERIALS, ELSEVIER SCIENCE PUBLISHERS BV., BARKING, GB, Bd. 32, Nr. 1, 1. Januar 2011 (2011-01-01) , Seiten 87-94, XP027493692, ISSN: 0142-9612, DOI: 10.1016/J.BIOMATERIALS.2010.08.090 [gefunden am 2010-09-24]
- V. VAIJAYANTHIMALA ET AL: "The long-term stability and biocompatibility of fluorescent nanodiamond as an in vivo contrast agent", BIOMATERIALS., Bd. 33, Nr. 31, 1. November 2012 (2012-11-01), Seiten 7794-7802, XP055518761, GB ISSN: 0142-9612, DOI: 10.1016/j.biomaterials.2012.06.084
- H Dieringa ET AL: "ECCM15 -15 TH EUROPEAN CONFERENCE ON COMPOSITE MATERIALS ULTRASONIC STIRRING AS A PRODUCTION PROCESS FOR NANOPARTICLE REINFORCED MAGNESIUM ALLOYS AND THE COMPRESSION CREEP RESPONSE OF ZE10 REINFORCED WITH CERIA NANOPARTICLES", , 24. Juni 2012 (2012-06-24), XP055519459, Gefunden im Internet: URL:http://www.escm.eu.org/eccm15/data/ass ets/1619.pdf [gefunden am 2018-10-26]
- SHERY L. Y. CHANG ET AL: "Counting vacancies and nitrogen-vacancy centers in detonation nanodiamond", NANOSCALE, Bd. 8, Nr. 20, 1. Januar 2016 (2016-01-01) , Seiten 10548-10552, XP055518970, United Kingdom ISSN: 2040-3364, DOI: 10.1039/C6NR01888B
- STEPAN V. BOLSHEDVORSKII ET AL: "Single bright NV centers in aggregates of detonation nanodiamonds", OPTICAL MATERIALS EXPRESS, Bd. 7, Nr. 11, 20. Oktober 2017 (2017-10-20), Seite 4038, XP055604089, DOI: 10.1364/OME.7.004038
- C. BRADAC ET AL: "Observation and control of blinking nitrogen-vacancy centres in discrete nanodiamonds", NATURE NANOTECHNOLOGY, Bd. 5, Nr. 5, 1. Mai 2010 (2010-05-01), Seiten 345-349, XP055105249, ISSN: 1748-3387, DOI: 10.1038/nnano.2010.56
- P. REINECK ET AL: "Bright and photostable nitrogen-vacancy fluorescence from unprocessed detonation nanodiamond", NANOSCALE, Bd. 9, Nr. 2, 1. Januar 2017 (2017-01-01), Seiten 497-502, XP055604227, United Kingdom ISSN: 2040-3364, DOI: 10.1039/C6NR07834F
- LANIN S N ET AL: "Modification of the surface chemistry of microdispersed sintered detonation nanodiamonds and its effect on the adsorption properties", ADSORPTION, SPRINGER US, NEW YORK, vol. 23, no. 5, 16 May 2017 (2017-05-16), pages 639-650, XP036257386, ISSN: 0929-5607, DOI: 10.1007/S10450-017-9883-4 [retrieved on 2017-05-16]
- Hajo Dieringa ET AL: "Magnesium Matrix Composites: State-of the-Art and what s the Future", Advanced Materials Research, vol. 410, 1 November 2011 (2011-11-01), pages 275-278, XP055519334, DOI: 10.4028/www.scientific.net/AMR.410.275
- TURCHENIUK K ET AL: "Biomedical applications of nanodiamond (Review)", NANOTECHNOLOGY, INSTITUTE OF PHYSICS PUBLISHING, BRISTOL, GB, vol. 28, no. 25, 1 June 2017 (2017-06-01), page 252001, XP020317269, ISSN: 0957-4484, DOI: 10.1088/1361-6528/AA6AE4 [retrieved on 2017-06-01]

## Beschreibung

### GEBIET DER ERFINDUNG

Die vorliegende Erfindung betrifft ein Implantatmaterial aus Magnesium oder einer Magnesiumlegierung und ein Verfahren zur Herstellung desselben.

### HINTERGRUND DER ERFINDUNG

Sowohl in der Veterinär- als auch in der Humanmedizin werden derzeit zur Versorgung von Frakturen gewichtstragender langer Röhrenknochen Metallimplantate aus medizinischem Stahl oder Titan verwendet. Diese Implantate sind in ihrem mechanischen Verhalten jedoch rigider als Knochen, was zum Phänomen der Belastungsabschirmung ("stress-shielding") führen kann. Aus diesen und anderen Gründen werden entsprechende Implantate in der Regel nach Funktionserfüllung wieder entfernt, was für den Patienten durch die notwendige Narkose und das erneute Gewebetrauma belastend sein kann.

Resorbierbare Implantate werden zunehmend zur Frakturversorgung interessant. Ziel ist es, dass die Implantate mit zunehmender Festigkeit des heilenden Knochens eine Belastungsanpassung durch langsame Abnahme ihre Stabilität erfahren. Der Einsatz der bisher zur Verfügung stehenden resorbierbaren Implantate aus unterschiedlichen Polymeren gelingt aufgrund ihrer niedrigen Festigkeiten am belasteten Knochen nicht optimal. Dagegen weisen Magnesium und seine Legierungen im Vergleich zu anderen metallischen Implantatmaterialien ein den Knochen ähnliches Elastizitätsmodul und günstige Zug-und Druckfestigkeit auf. Magnesium und seine Legierungen besitzen höhere Festigkeiten und einen größeren Elastizitätsmodul als resorbierbare Polymere und stehen daher im Fokus der wissenschaftlichen Forschung. Bioresorbierbare Implantate, insbesondere aus Magnesium oder einer Magnesiumlegierung zur Behandlung von Knochenfrakturen sind beispielsweise aus der EP 2 318 057 B1 und darin zitierten Druckschriften oder der DE 10 2005 060 203 A1 bekannt.

Resorbierbare Implantate werden nicht nur zur Frakturversorgung eingesetzt. Heutzutage werden Implantate aus Magnesium und seinen Legierungen besonders häufig als Stents eingesetzt, die zur Behandlung von Stenosen (Gefäßverengungen) dienen. Stents weisen ein rohrförmiges oder hohlzylinderförmiges Grundgitter auf, dass an beiden Längsenden offen ist. Das rohrförmige Grundgitter einer derartigen Endoprothese wird in das zu behandelnde Gefäß eingesetzt und dient dazu, das Gefäß zu stützen. Biodegradable Stents aus Magnesium oder Magnesiumlegierung sind beispielsweise aus der EP 2 198 898 B1 und darin zitierten Druckschriften bekannt.

Nachteil der bekannten Implantate ist jedoch, dass deren Resorption bisher nur mittels Röntgen- oder CT-Untersuchungen verfolgt werden kann. Diese Untersuchungen sind vergleichsweise aufwendig und kostenintensiv. Aufgabe der vorliegenden Erfindung ist es, ein Implantatmaterial aus Magnesium oder einer Magnesiumlegierung und ein Verfahren zur Herstellung desselben zur Verfügung zu stellen, dessen Resorption im Körper des Patienten auf einfache Weise verfolgt werden kann, ohne dass Röntgen- oder CT-Untersuchungen erforderlich sind.

WO2013158869 beschreibt Kompositmaterial für medizinische Implantate, enthaltend eine Magnesiummatrix mit homogen eingelagerten, die Korrosion hemmenden Nano-Diamanten die insbesondere durch Detonationsverfahren hergestellt sind.

Q.ZHANG et al. ["Fluorescent PLLA-nanodiamond composites for bone tissue engineering",BIOMATERIALS, Bd. 32 (1), 2011, 87-94, XP027493692] beschreiben PLLA-Knochenscaffold-Zusammensetzungen, enthaltend mit Octadecylamine funktionalisierte Nanodiamanten die die Härte erhöhen und Fluoreszenz zeigen.

### ZUSAMMENFASSUNG DER ERFINDUNG

Gelöst wird die Aufgabe durch ein resorbierbares Implantatmaterial gemäß Anspruch 1, das homogen verteilt fluoreszierende Nanodiamanten mit Stickstoff-Vakanzzentren in einer Matrix aus Magnesium oder einer Magnesiumlegierung umfasst, wobei die fluoreszierenden

Nanodiamanten Stickstoff-Vakanz-Zentren (NV-Zentren) mit einer detektierbaren Fluoreszenzbande nach Anregung durch einen 532 nm-Laserstrahl umfassen, die bei einer Wellenlänge zwischen 650 nm und 700 nm zentriert ist, zur Verwendung bei der Frakturversorgung oder zur Behandlung von Stenosen, wobei die Resorption des Implantatmaterials im Körper des Patienten mittels Fluoreszenzspektroskopie des Blutplasmas detektiert wird.

Gelöst wird die Aufgabe auch durch ein Verfahren zur Herstellung eines Implantatmaterials gemäß Anspruch 7, bei dem Magnesium oder eine Magnesiumlegierung aufgeschmolzen wird, zu der Schmelze fluoreszierende Nanodiamanten mit Stickstoff-Vakanzzentren gegeben werden und die mit Nanodiamanten versehene Schmelze aus Magnesium oder einer Magnesiumslegierung einer Ultraschallbehandlung unterzogen wird, sowie durch ein Verfahren gemäß Anspruch 14.

### DETAILLIERTE BESCHREIBUNG DER ERFINDUNG

Floureszierende Nanodiamanten (FNDs) sind beispielsweise aus K. Merchand und S.K. Sarkar, IEEE JOURNAL OF SELECTED TOPICS IN QUANTUM ELECTRONICS, VOL. 22, NO. 3, MAY/JUNE 2016 als Proteinmarker bekannt. Diese werden bislang in der Forschung zum Sichtbarmachen von biologischen Zellprozessen eingesetzt. Floureszierende Nanodiamanten sind für den Organismus unbedenklich und bieten aufgrund von Stickstoff-Vakanz-Zentren (NV-Zentren) eine stabile Emission im nahen Infrarotbereich. Das NV-Zentrum im Diamant wird von einem Stickstoffatom, das ein Kohlenstoffatom im Diamantgitter substituiert, und einer direkt benachbarten Leerstelle im Diamantgitter gebildet. Diese Vakanz ermöglicht es den NV-Zentren, eine negative (NV⁻-Zentrum) oder neutrale (NV⁰-Zentrum) Ladung anzunehmen. Das NV-Zentrum im Diamant besitzt herausragende photophysikalische Eigenschaften.

Im Kontext des Bioimaging ist die herausragende Eigenschaft des negativ geladenen NV⁻-Zentrum, dass es zwischen 480 nm und 580 nm optisch angeregt werden kann, um eine breite Lumineszenzbande zu emittieren, die bei etwa 700 nm zentriert ist. Die angeregte Zustandslebensdauer beträgt ungefähr 17 ns, wie in J.M. Say, "Luminescent Nanodiamonds for Biomedical Applications", Biophys. Rev. (2011) 3:171-184 beschrieben ist. Das neutrale NV⁰-Zentrum emittiert mit einer relativ scharfen Linie bei 637 nm.

Die Herstellung von fluoreszierenden Nanodiamanten mit Stickstoff-Leerstellen ist beispielsweise in der US 2014/0065424 A1 oder der WO 2017/108655 A1 beschrieben, auf die hier vollständig Bezug genommen wird.

Zur Herstellung der fluoreszierenden Nanodiamanten mit Stickstoff-Vakanz-Zentren muss sein Kristallgitter unter kontrollierten Bedingungen beschädigt werden, um diese Stickstoff-Vakanz-Zentren zu bilden, die erst eine optische Bildgebung ermöglichen. Am häufigsten geschieht dies durch die Bestrahlung von Nanodiamanten mit schnellen Ionen in Teilchenbeschleunigern, so auch in den Verfahren gemäß US 2014/0065424 A1 mit einem Elektronenstrahl bei einer Beschleunigungsenergie zwischen 7 MeV und 15 MeV. Diese beschleunigten Ionen sind in der Lage, Kohlenstoffatome aus dem Kristallgitter eines Nanodiamanten herauszuschlagen und hinterlassen dabei Löcher, die als Vakanzen bezeichnet werden und bei hohen Temperaturen mit den im Kristall vorhandenen Stickstoffatomen als Verunreinigungen gekoppelt sind. Anstelle einer teuren und zeitaufwendigen Bestrahlung in einem Beschleuniger nutzen neuere Verfahren die Bestrahlung in einem Kernreaktor, die viel schneller und weitaus kostengünstiger ist. Dazu müssen die Nanokristalle zunächst in geschmolzenem Boroxid dispergiert und dann in einem Kernreaktor einer Neutronenbestrahlung ausgesetzt werden. Der Neutroneneinfang durch Borkerne erzeugt einen dichten Regen aus Helium- und Lithiumionen, die in den Nanokristallen die gleiche Wirkung haben wie die in einem Beschleuniger erzeugten Ionen: die kontrollierte Erzeugung von Kristalldefekten.

Gemäß einer Ausführungsform der vorliegenden Erfindung, weisen die fluoreszierenden Nanodiamanten eine Konzentration an Stickstoff-Vakanz-Zentren von mehr als 10 ppm, bevorzugt mehr als 20 ppm, bevorzugter mehr als 25 ppm auf, bestimmt mittels Epifluoreszenz nach Anregung durch einen 532 nm-Laserstrahl. Die Methode zur Bestimmung der Konzentration an Stickstoff-Vakanz-Zentren in einem Nanodiamanten mittels Epifluoreszenz nach Anregung durch einen 532 nm-Laserstrahl wird beispielsweise in Chi-Chen Fu et al. "Characterization ans application of single fluorescent nanodiamonds as cellular biomarkers", PNAS, Vol. 194, Nr. 3, 727-732 (2007) beschrieben, auf die hier vollständig Bezug genommen wird. Die Konzentration an Stickstoff-Vakanz-Zentren wird insbesondere durch Vergleich der Intensität der gemessenen Fluoreszenz mit der Intensität der Fluoreszenz von "Single-Defect"-Diamanten bestimmt (siehe Literaturangaben in Chi-Chen Fu et al.). Derartige Angaben sind beispielsweise in F. Treussart et al. (2006) Physica B Condensed Matter 376:926-929 veröffentlicht.

Fluoreszierende Nanodiamanten, die diese Eigenschaften erfüllen, sind beispielsweise auch von der Firma Sigma-Aldrich Chemie GmbH, Steinheim, Deutschland, kommerziell erhältlich.

Zur Untersuchung der Transmissionseigenschaften eignet sich jegliche Lichtquelle, die in der Lage ist, die NV-Zentren zu aktivieren, z.B. ein Helium-Neon-Laser dessen Wellenlänge von 632,7 nm ungefähr der ZPL des NV-Zentrums entspricht oder ein Solid-State Laser (JL-LD532-GTE; Jetlser), der bei einer Wellenlänge von 532 nm betrieben wird.

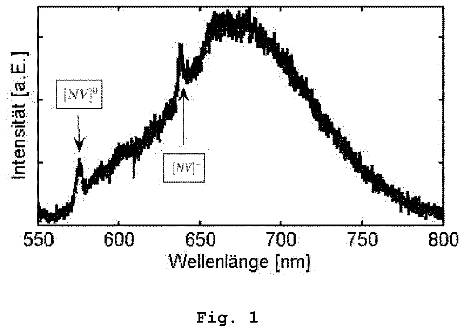

**Figur 1** zeigt das Fluoreszenzspektrum eines NV⁻-Zentrums in einem Nanodiamanten bei Raumtempertaur, angeregt durch einen 532 nm-Laserstrahl. Die Figur zeigt eine breite Fluoreszenzbande, die bei einer Wellenlänge von etwa 700 nm zentriert ist.

Das erfindungsgemäße Implantatmaterial aus Magnesium oder einer Magnesiumlegierung, das homogen verteilt fluoreszierende Nanodiamanten enthält, kann durch Gießen hergestellt werden. Danach kann es stranggepresst werden oder mittels pulvermetallurgischer Verfahren wie der MIM-Technologie zu Implantatkörpern verarbeitet werden. Mit der Resorption des Implantatmaterials im Körper des Patienten gelangen die fluoreszierenden Nanodiamanten in den Blutkreislauf, wo sie mittels Fluoreszenzspektroskopie oder auf andere Weise nachgewiesen werden können. Die fluoreszierenden Nanodiamanten werden nach und nach aus dem Körper ausgeschieden. Auf diese Weise erhält man ein An-/Abflutungsprofil im Plasma, das nach Kalibrierung Rückschlüsse auf die Resorption des Implantatmaterials zulässt.

Sofern eine Magnesiumlegierung als Matrixmaterial verwendet wird, werden bevorzugt Legierungselemente verwendet, die als nicht gesundheitsgefährdend gelten. Bevorzugt werden Magnesiumlegierungen mit Legierungselementen verwendet, die ausgewählt sind aus der Gruppe bestehend aus Lithium, Calcium, Kalium, Strontium, Barium, Scandium, Yttrium, Lanthan, Praseodym, Neodym, Samarium, Europium, Gadolinium, Dysprosium, Silizium, Kupfer, Zink, Gallium, Gold, Silber, Bismut, Eisen und Kombinationen davon. Bevorzugter werden Magnesiumlegierungen verwendet, wie sie in DE 10 2016 007 176 A1, oder DE 10 2016 119 227 A1 beschrieben sind, auf die hier vollständig Bezug genommen wird.

Erfindungsgemäß wird das Implantatmaterial hergestellt, indem Magnesium oder eine Magnesiumlegierung aufgeschmolzen wird, zu der Schmelze Nanodiamanten gegeben werden und die mit Nanodiamanten versehene Schmelze aus Magnesium oder einer Magnesiumlegierung einer Ultraschallbehandlung unterzogen wird.

Ein derartiges Verfahren zur homogenen Verteilung von Nanopartikeln in einer Schmelze aus Magnesium oder einer Magnesiumlegierung wird in dem Artikel von H. Dieringa et al. "Ultrasound Assisted Casting of an AM60 Based Metal Matrix Nanocomposite, Its Properties, and Recyclability" in Metals 2017, 7, 338 beschrieben, auf den hier vollständig Bezug genommen wird.

Bei einem bevorzugten Verfahren zur Herstellung des erfindungsgemäßen Implantatmaterials wird Magnesium oder eine Magnesiumlegierung bevorzugt in einem ersten Schritt in einer in einem Ofen befindlichen Kokille unter Schutzgas und unter Rühren aufgeschmolzen, in einem zweiten Schritt wird die Schmelze mit den Nanodiamanten versetzt und in einem dritten Schritt werden die in die Schmelze eingebrachten Nanodiamanten mittels einer Sonotrode dispergiert und deagglomeriert. Es ist ferner bevorzugt, dass die Kokille mit der Schmelze nach Herausnehmen des Rührers und der Sonotrode in ein Wasserbad getaucht wird. Somit erfolgt eine Erstarrung der Schmelze von "unten nach oben", wodurch eine Lunkerbildung vermieden wird.

Das so hergestellte Implantatmaterial kann im Anschluss auf gewöhnliche Weise weiterverarbeitet werden. Beispielsweise kann das Implantatmaterial wieder aufgeschmolzen und anschließend in die gewünschte Form zur Bildung eines Implantatkörpers gegossen werden. Das Material kann auch stranggepresst werden, um aus dem Extrudat Implantate zu fertigen. Alternativ kann das Implantatmaterial zu Pulver weiterverarbeitet und mittels Metallspritzgiessen ("metal injection moulding", MIM) zu einem Implantatkörper weiterverarbeitet werden.

Das erfindungsgemäße Implantatmaterial umfasst bevorzugt homogen verteilte fluoreszierende Nanodiamanten in einer Matrix aus Magnesium oder einer Magnesiumlegierung in einer Menge von 0,1 bis 5 Gew.-%, bevorzugt 0,5 bis 1,5 Gew.-% auf Basis des Gewichts an Magnesium bzw. Magnesiumlegierung. Die Nanodiamanten haben bevorzugt eine Teilchengröße von 1 bis 20 nm, bevorzugt 3 bis 8 nm.

Wie beschrieben wird zur Herstellung des erfindungsgemäßen Implantatmaterials das Magnesium oder die Magnesiumlegierung bevorzugt in einem ersten Schritt in einer in einem Ofen befindlichen Kokille unter Schutzgas und unter Rühren aufgeschmolzen, wie in H. Dieringa et al. "Ultrasound Assisted Casting of an AM60 Based Metal Matrix Nanocomposite, Its Properties, and Recyclability" in Metals 2017, 7, 338 beschrieben. Die Schmelze wird vorzugsweise mechanisch gerührt, vorzugsweise bei 150 bis 250 UpM. Anschließend werden die fluoreszierenden Nanodiamanten der Schmelze zugefügt. Nach Zugabe der fluoreszierenden Nanodiamanten wird die Schmelze mit Ultraschall behandelt. Dazu wird bevorzugt eine Sonotrode in die Schmelze eingebracht. Die Ultraschallbehandlung findet bevorzugt über eine Zeitspanne von 1 Min. bis 10 Min., bevorzugter 2 Min. bis 5 Min. statt.

Es ist bevorzugt, dass nach dem Mischen und der Ultraschallbehandlung der Rührer und die Sonotrode entfernt werden und die Kokille langsam aus dem Ofen in ein Wasserbad abgesenkt wird, wo die Schmelze erstarrt.

Das so hergestellte Implantatmaterial kann im Anschluss auf gewöhnliche Weise weiterverarbeitet werden. Beispielsweise kann das Implantatmaterial wieder aufgeschmolzen und anschließend in die gewünschte Form gegossen werden, um einen metallischen Implantatkörper zu liefern. Das erfindungsgemäße Implantatmaterial kann auch stranggepresst werden und aus dem Extrudat kann ein Implantatkörper gefertigt werden.

Das erfindungsgemäße Implantatmaterial kann auch mit Hilfe der MIM-Technologie zu einem metallischen Implantatkörper verarbeitet werden. Mit Hilfe der MIM-Technologie lassen sich kleine, komplexe und präzise geformte Metallbauteile endformnah fertigen. Die MIM-Technologie gehört zu den sogenannten pulvermetallurgischen Verfahren, bei denen kein massiver Metallkörper, sondern feines Metallpulver als Ausgangsmaterial für das herzustellende Bauteil verwendet wird. MIM steht dabei für "Metal Injection Moulding" (Metallpulverspritzguss). Beim MIM-Verfahren wird das Metallpulver durch Zusatz von thermoplastischen Bindemitteln fließfähig gemacht und das fließfähige Gemisch in eine Spritzgussform eingebracht. Nach der Formgebung wird der Bindemittelanteil wieder entfernt und das Bauteil gesintert. Magnesiumbauteile können mithilfe der MIM-Technologie nach dem in M. Wolff et. al. "Magnesium powder injection moulding for biomedical application", Powder Metallurgy, 2014 (Vol. 57, No. 5), 331-340 beschriebenen Verfahren hergestellt werden, auf das hier vollständig Bezug genommen wird.

Das Bindemittel sorgt bei Anwendung der MIM-Technologie für eine temporäre Verbindung während des Urformens bzw. der Formgebung und sichert die Stabilität des Bauteils bis zur endgültigen Kompaktierung des Metallpulvers durch Sintern. Ein Teil des Bindemittels wird in der Regel bereits vor der Sinterung, zum Beispiel Mithilfe eines Lösungsmittels entfernt (Lösemittelentbinderung). Der Rest des Bindemittels zersetzt sich bei der thermischen Entbinderung bei Temperaturen von etwa 300°C bis 500°C und entweicht gasförmig.

## Patentansprüche

1. Resorbierbares Implantatmaterial, das homogen verteilt fluoreszierende Nanodiamanten mit Stickstoff-Vakanz-Zentren in einer Matrix aus Magnesium oder einer Magnesiumlegierung umfasst, wobei die fluoreszierenden Nanodiamanten Stickstoff-Vakanz-Zentren (NV-Zentren) mit einer detektierbaren Fluoreszenzbande nach Anregung durch einen 532 nm-Laserstrahl umfassen, die bei einer Wellenlänge zwischen 650 nm und 700 nm zentriert ist, zur Verwendung bei der Frakturversorgung oder zur Behandlung von Stenosen, wobei die Resorption des Implantatmaterials im Körper des Patienten mittels Fluoreszenz-spektroskopie des Blutplasmas detektiert wird.

2. Implantatmaterial zur Verwendung nach Anspruch 1, wobei die fluoreszierenden Nanodiamanten eine Konzentration an Stickstoff-Vakanz-Zentren von mehr als 10 ppm, bevorzugt mehr als 20 ppm, bestimmt mittels Epifluoreszenz nach Anregung durch einen 532 nm-Laserstrahl, aufweisen.

3. Implantatmaterial zur Verwendung nach Anspruch 1 oder 2, wobei die homogen verteilten fluoreszierenden Nanodiamanten in einer Menge von 0,1 bis 5 Gew.-% auf Basis des Gewichts des Magnesiums bzw. der Magnesiumlegierung in der Matrix vorliegen.

4. Implantatmaterial zur Verwendung nach Anspruch 3, wobei die homogen verteilten fluoreszierenden Nanodiamanten in einer Menge von 0,5 bis 1,5 Gew.-% auf Basis des Gewichts des Magnesiums bzw. der Magnesiumlegierung in der Matrix vorliegen.

5. Implantatmaterial zur Verwendung nach einem der vorgehenden Ansprüche, wobei die fluoreszierenden Nanodiamanten eine Teilchengröße von Teilchengröße von 1 bis 20 nm aufweisen.

6. Implantatmaterial zur Verwendung nach Anspruch 5, wobei die fluoreszierenden Nanodiamanten eine Teilchengröße von Teilchengröße von 3 bis 8 nm aufweisen.

7. Verfahren zur Herstellung eines Implantatmaterials gemäß einem der vorgehenden Ansprüche, bei dem Magnesium oder eine Magnesiumlegierung aufgeschmolzen wird, zu der Schmelze Nanodiamanten gegeben werden und die mit Nanodiamanten versehene Schmelze aus Magnesium oder einer Magnesiumslegierung einer Ultraschallbehandlung unterzogen wird.

8. Verfahren nach Anspruch 7, bei dem das Magnesium oder die Magnesiumlegierung in einem ersten Schritt in einer in einem Ofen befindlichen Kokille unter Schutzgas und unter Rühren aufgeschmolzen wird, die Schmelze mechanisch gerührt wird, anschließend die fluoreszierenden Nanodiamanten der Schmelze zugefügt werden und nach Zugabe der fluoreszierenden Nanodiamanten wird die Schmelze mit Ultraschall behandelt wird.

9. Verfahren nach einem der Ansprüche 7 bis 8, bei dem die Ultraschallbehandlung über eine Zeitspanne von 1 Min. bis 10 Min. stattfindet.

10. Verfahren nach einem der Ansprüche 7 bis 9, bei dem die Kokille nach der Ultraschallbehandlung in ein Wasserbad überführt wird, wo die Schmelze erstarrt.

11. Verfahren nach einem der Ansprüche 7 bis 10, bei dem das Implantatmaterial wieder aufgeschmolzen und anschließend in die gewünschte Form gegossen werden, um ein metallisches Implantat zu liefern.

12. Verfahren nach einem der Ansprüche 7 bis 11, bei dem das Implantatmaterial extrudiert wird und das Extrudat als Grundstoff für die Fertigung eines Implantates dient.

13. Verfahren nach einem der Ansprüche 7 bis 12, bei dem das Implantatmaterial mittels MIM-Technologie in ein metallisches Implantat überführt wird.

14. Verfahren zur Detektion der Resorption eines resorbierbaren Implantatmaterials mittels Fluoreszenzspektroskopie des Blutplasmas im des Patienten nach Anregung durch einen 532 nm-Laserstrahl, wobei ein Implantatmaterial, das homogen verteilt fluoreszierende Nanodiamanten mit Stickstoff-Vakanz-Zentren in einer Matrix aus Magnesium oder einer Magnesiumlegierung umfasst, verwendet wird, wobei die fluoreszierenden Nanodiamanten Stickstoff-Vakanz-Zentren (NV-Zentren) mit einer detektierbaren Fluoreszenzbande nach Anregung durch einen 532 nm-Laserstrahl umfassen, die bei einer Wellenlänge zwischen 650 nm und 700 nm zentriert ist.

## Claims

1. A resorbable implant material comprising homogeneously distributed fluorescent nanodiamonds having nitrogen-vacancy centers in a matrix of magnesium or a magnesium alloy, wherein the fluorescent nanodiamonds have nitrogen-vacancy centers (NV centers) which after excitation by a 532 nm laser beam have a detectable fluorescence band centered at a wavelength between 650 nm and 700 nm, for use in the treatment of fractures or for treatment of stenoses, wherein the resorption of the implant material in the patient's body is detected by means of fluorescence spectroscopy of the blood plasma.

2. The implant material for use according to claim 1, wherein the fluorescent nanodiamonds have a concentration of nitrogen-vacancy centers of more than 10 ppm, preferably more than 20 ppm, determined by epifluorescence after excitation by a 532 nm laser beam.

3. The implant material for use according to claim 1 or 2, wherein the homogeneously distributed fluorescent nanodiamonds are present in an amount of 0.1% to 5% by weight based on the weight of magnesium/of the magnesium alloy in the matrix.

4. The implant material for use according to claim 3, wherein the homogeneously distributed fluorescent nanodiamonds are present in an amount of 0.5% to 1.5% by weight based on the weight of the magnesium/of the magnesium alloy in the matrix.

5. The implant material for use according to any of the preceding claims, wherein the fluorescent nanodiamonds have a particle size of 1 to 20 nm.

6. The implant material for use according to claim 5, wherein the fluorescent nanodiamonds have a particle size of 3 to 8 nm.

7. A method for producing an implant material according to any of the preceding claims, wherein magnesium or a magnesium alloy is melted, nanodiamonds are added to the melt and the melt of magnesium or a magnesium alloy provided with nanodiamonds is subjected to an ultrasound treatment.

8. The method according to claim 7, wherein the magnesium or the magnesium alloy is melted in a permanent mold in a furnace under protective gas and with stirring in a first step, the melt is stirred mechanically, the fluorescent nanodiamonds are then added to the melt and after addition of the fluorescent nanodiamonds the melt is treated with ultrasound.

9. The method according to any of claims 7 to 8, wherein the ultrasound treatment is carried out over a period of 1 min to 10 min.

10. The method according to any of claims 7 to 9, wherein after the ultrasound treatment the mold is transferred into a water bath where the melt solidifies.

11. The method according to any of claims 7 to 10, wherein the implant material is remelted and subsequently poured into the desired mold to afford a metallic implant.

12. The method according to any of claims 7 to 11, wherein the implant material is extruded and the extrudate serves as a precursor for fabrication of an implant.

13. The method according to any of claims 7 to 12, wherein the implant material is converted into a metallic implant using MIM technology.

14. A method for detecting the resorption of a resorbable implant material in the patient's body by means of fluorescence spectroscopy of the blood plasma, after excitation by a 532 nm laser beam, wherein an implant material, comprising homogeneously distributed fluorescent nanodiamonds having nitrogen-vacancy centers in a matrix of magnesium or a magnesium alloy, is used, wherein the fluorescent nanodiamonds have nitrogen-vacancy centers (NV centers) which after excitation by a 532 nm laser beam have a detectable fluorescence band centered at a wavelength between 650 nm and 700 nm.

## Revendications

1. Matériau d'implant résorbable, qui comprend des nanodiamants fluorescents répartis de manière homogène avec des centres azote-lacune dans une matrice de magnésium ou d'un alliage de magnésium, dans lequel les nanodiamants fluorescents comprennent des centres azote-lacune (centres NV) avec une bande fluorescente détectable après excitation par un faisceau laser de 532 nm, qui est centrée pour une longueur d'onde comprise entre 650 nm et 700 nm, pour une utilisation pour le traitement de fracture ou pour les soins apportés aux sténoses, dans lequel la résorption du matériau d'implant dans le corps du patient est détectée au moyen d'une spectroscopie de fluorescence du plasma sanguin.

2. Matériau d'implant pour une utilisation selon la revendication 1, dans lequel les nanodiamants fluorescents présentent une concentration de centres azote-lacune de plus de 10 ppm, de préférence de plus de 20 ppm, déterminée au moyen d'une épifluorescence après excitation par un faisceau laser de 532 nm.

3. Matériau d'implant pour une utilisation selon la revendication 1 ou 2, dans lequel les nanodiamants fluorescents répartis de manière homogène sont présents dans une quantité de 0,1 à 5 % en poids sur la base du poids du magnésium ou de l'alliage de magnésium dans la matrice.

4. Matériau d'implant pour une utilisation selon la revendication 3, dans lequel les nanodiamants fluorescents répartis de manière homogène sont présents dans une quantité de 0,5 à 1,5 % en poids sur la base du poids du magnésium ou de l'alliage de magnésium dans la matrice.

5. Matériau d'implant pour une utilisation selon l'une quelconque des revendications précédentes, dans lequel les nanodiamants fluorescents présentent une taille de particule d'une taille de particule de 1 à 20 nm.

6. Matériau d'implant pour une utilisation selon la revendication 5, dans lequel les nanodiamants fluorescents présentent une taille de particule d'une taille de particule de 3 à 8 nm.

7. Procédé pour la fabrication d'un matériau d'implant selon l'une quelconque des revendications précédentes, selon lequel du magnésium ou un alliage de magnésium est fondu, des nanodiamants sont ajoutés à la matière fondue et la matière fondue de magnésium ou d'un alliage de magnésium pourvue de nanodiamants est soumise à un traitement par ultrasons.

8. Procédé selon la revendication 7, selon lequel le magnésium ou l'alliage de magnésium est fondu dans une première étape dans une lingotière se trouvant dans un four sous gaz protecteur et sous agitation, la matière fondue est agitée mécaniquement, puis les nanodiamants fluorescents sont ajoutés à la matière fondue et après addition des nanodiamants fluorescents la matière fondue est traitée avec des ultrasons.

9. Procédé selon l'une quelconque des revendications 7 à 8, selon lequel le traitement par ultrasons a lieu pendant un laps de temps de 1 min à 10 min.

10. Procédé selon l'une quelconque des revendications 7 à 9, selon lequel la lingotière après le traitement par ultrasons est amenée dans un bain-marie, où la matière fondue se solidifie.

11. Procédé selon l'une quelconque des revendications 7 à 10, selon lequel le matériau d'implant est à nouveau fondu et ensuite coulé dans la forme souhaitée, afin de fournir un implant métallique.

12. Procédé selon l'une quelconque des revendications 7 à 11, selon lequel le matériau d'implant est extrudé et l'extrudat sert de substance de base pour la fabrication d'un implant.

13. Procédé selon l'une quelconque des revendications 7 à 12, selon lequel le matériau d'implant est amené dans un implant métallique au moyen d'une technologie MIM.

14. Procédé pour la détection de la résorption d'un matériau d'implant résorbable au moyen d'une spectroscopie de fluorescence du plasma sanguin dans le corps du patient après excitation par un faisceau laser de 532 nm, dans lequel un matériau d'implant, qui comprend des nanodiamants fluorescents répartis de manière homogène avec des centres azote-lacune dans une matrice de magnésium ou d'un alliage de magnésium, est utilisé, dans lequel les nanodiamants fluorescents comprennent des centres azote-lacune (centres NV) avec une bande fluorescente détectable après excitation par un faisceau laser de 532 nm, qui est centrée pour une longueur d'onde comprise entre 650 nm et 700 nm.
